# EUROPEAN PATENT SPECIFICATION

(11) **EP 2 928 439 B1**
(45) Date of publication and mention of the grant of the patent: **19.07.2017**
(21) Application number: 12799707.0
(22) Date of filing: 05.12.2012
(51) Int. Cl.: A61K 8/27, A61Q 11/00

(54) **ZINC PHOSPHATE CONTAINING COMPOSITIONS**
ZINKPHOSPHATHALTIGE ZUSAMMENSETZUNGEN
COMPOSITIONS CONTENANT DU PHOSPHATE DE ZINC

(43) Date of publication of application: 14.10.2015
(60) Divisional application: 17174701.7
(73) Proprietor: Colgate-Palmolive Company, New York, NY 10022 (US)
(72) Inventor: REGE, Aarti, East Windsor, New Jersey 08520 (US); SURIANO, David F., Edison, New Jersey 08817 (US); SULLIVAN, Richard, Atlantic Highlands, New Jersey 07716 (US); STRANICK, Michael A., Bridgewater, New Jersey 08807 (US)
(74) Representative: Wibbelmann, Jobst
(86) International application number: PCT/US2012/068000
(87) International publication number: WO 2014/088573

(56) References cited:
- WO-A1-2010/112577
- US-A1- 2008 181 856
- US-B1- 6 221 340
- DATABASE WPI Week 200616 Thomson Scientific, London, GB; AN 2006-150515 XP002712510, & JP 2006 045215 A (TAISHO PHARM CO LTD) 16 February 2006 (2006-02-16)

## Description

### BACKGROUND

Dental erosion involves demineralization and damage to the tooth structure due to acid attack from nonbacterial sources. Erosion is found initially in the enamel and, if unchecked, may proceed to the underlying dentin. Dental erosion may be caused or exacerbated by acidic foods and drinks, exposure to chlorinated swimming pool water, and regurgitation of gastric acids. The tooth enamel is a negatively charged surface, which naturally tends to attract positively charged ions such as hydrogen and calcium ions, while resisting negatively charged ions such as fluoride ions. Depending upon relative pH of surrounding saliva, the tooth enamel will lose or gain positively charged ions such as calcium ions. Generally saliva has a pH between 7.2 to 7.4. When the pH is lowered and concentration of hydrogen ions becomes relatively high, the hydrogen ions will replace the calcium ions in the enamel, forming hydrogen phosphate (phosphoric acid), which damages the enamel and creates a porous, sponge-like roughened surface. If saliva remains acidic over an extended period, then remineralization may not occur, and the tooth will continue to lose minerals, causing the tooth to weaken and ultimately to lose structure.

There is a need for improved products for treating and reducing erosion.

Heavy metal ions, such as zinc, are resistant to acid attack. Zinc ranks above hydrogen in the electrochemical series, so that metallic zinc in an acidic solution will react to liberate hydrogen gas as the zinc passes into solution to form di-cations, Zn²⁺. Zinc has been shown to have antibacterial properties in plaque and caries studies.

Soluble zinc salts, such as zinc citrate, have been used in dentifrice compositions, but have several disadvantages. Zinc ions in solution impart an unpleasant, astringent mouthfeel, so formulations that provide effective levels of zinc, and also have acceptable organoleptic properties, have been difficult to achieve. Moreover, free zinc ions may react with fluoride ions to produce zinc fluoride, which is insoluble and so reduces the availability of both the zinc and the fluoride. Finally, the zinc ions will react with anionic surfactants such as sodium lauryl sulfate, thus interfering with foaming and cleaning.

Zinc phosphate (Zn₃(PO₄)₂) is insoluble in water, although soluble in acidic or basic solutions, e.g., solutions of mineral acids, acetic acid, ammonia, or alkali hydroxides. *See, e.g.,* Merck Index, 13th Ed. (2001) p. 1812, monograph number 10205. Partly because it is viewed in the art as a generally inert material, it is commonly used in dental cements, for example in cementation of inlays, crowns, bridges, and orthodontic appliances, which are intended to endure in the mouth for many years. Zinc phosphate dental cements are generally prepared by mixing zinc oxide and magnesium oxide powders with a liquid consisting principally of phosphoric acid, water, and buffers, so the cement comprising zinc phosphate is formed *in situ* by reaction with phosphoric acid.

### SUMMARY

It has now been discovered that zinc phosphate, when placed in formulation, e.g., at acidic or basic pH, can dissolve sufficiently upon use to provide an effective concentration of zinc ions to the enamel, thereby protecting against erosion, reducing bacterial colonization and biofilm development, and providing enhanced shine to the teeth. The formulation invariably comprises arginine (a basic amino acid), which can confer a basic pH to the formulation. It has also been discovered that zinc phosphate in a formulation with a second phosphate source enhances phosphate deposition. This is all unexpected, in view of the poor solubility of zinc phosphate, and the art-recognized view that it is substantially inert in conditions in the oral cavity, as evidenced by its widespread use in dental cement. At the same time, the formulations containing zinc phosphate do not exhibit the poor taste and mouthfeel, poor fluoride delivery, and poor foaming and cleaning associated with conventional zinc-based oral care products, which use more soluble zinc salts.

The invention thus provides a dentifrice according to claim 1 comprising (i) zinc phosphate, and (ii) arginine in free or orally acceptable salt form in an amount of 0.5 to 10 % by weight, wherein the zinc phosphate is added to the dentifrice as a preformed salt in an amount of 0.5 to 4 % by weight. Preferred features are defined in the dependent claims. The compositions may optionally further comprise a fluoride source and or an additional phosphate source. The compositions may be formulated in a conventional dentifrice base, e.g., comprising abrasives, e.g., silica abrasives, surfactants, foaming agents, vitamins, polymers, enzymes, humectants, thickeners, antimicrobial agents, preservatives, flavorings, colorings, and/or combinations thereof. For example, in one embodiment, the invention provides a dentifrice comprising ca. 2% zinc phosphate, ca. 5% arginine, ca. 5% alkali phosphate salts, and ca. 1450ppm fluoride, in a silica abrasive dentifrice base.

The invention also provides a mouthwash according to claim 7. Preferred features are defined in the dependent claims.

Also described are methods of using the compositions of the invention to reduce and inhibit acid erosion of the enamel, clean the teeth, reduce bacterially-generated biofilm and plaque, reduce gingivitis, inhibit tooth decay and formation of cavities, and reduce dentinal hypersensitivity, comprising brushing the teeth with a composition of the invention.

Further areas of applicability of the present invention will become apparent from the detailed description provided hereinafter.

### DETAILED DESCRIPTION

The following description of the preferred embodiment(s) is merely exemplary in nature and is in no way intended to limit the invention, its application, or uses.

As used herein, the term "preformed salt" - when used in reference to zinc phosphate - means that the zinc phosphate is not formed in situ in the dentifrice or mouthwash, e.g. through the reaction of phosphoric acid and a zinc salt.

The disclosure therefore provides, in a first embodiment, an oral care composition for intermittent use, e.g., daily use, e.g., in the form of a dentifrice or mouthwash, comprising zinc phosphate (Composition 1), e.g.,
1.1. Composition 1 in the form of a dentifrice comprising zinc phosphate in a dentifrice base, e.g., wherein the zinc phosphate is present in an effective amount, e.g., in an amount of 0.5 to 4% by weight, e.g., about 1 to 3% by weight, in a dentifrice base.
1.2. Composition 1.1, wherein the dentifrice base comprises an abrasive, e.g., an effective amount of a silica abrasive, e.g., 10-30%, e.g., about 20%.
1.3. Composition 1 in the form of a mouthwash comprising zinc phosphate, e.g., in an amount of 0.005 - 0.05% by weight, e.g., about 0.01 - 0.03% by weight in a mouthwash base.
1.4. Any of the foregoing compositions further comprising an effective amount of a fluoride ion source, e.g., providing 500 to 3000 ppm fluoride.
1.5. Any of the foregoing compositions further comprising an effective amount of fluoride, e.g., wherein the fluoride is a salt selected from stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.
1.6. Any of the foregoing compositions comprising an amino acid in an amount sufficient to enhance the solubility of the zinc phosphate, e.g. about 0.5 wt. % to about 20 wt. % of the total composition weight, about 0.5 wt. % to about 10 wt. % of the total composition weight, for example about 1.5 wt. %, about 3.75 wt. %, about 5 wt. %, or about 7.5 wt. % of the total composition weight in the case of a dentifrice, or for example about 0.5-2 wt. %, e.g., about 1% in the case of a mouthwash.
1.7. Any of the foregoing compositions comprising a basic amino acid, e.g., arginine or lysine or combinations thereof, for example 1-arginine, e.g in an effective amount e.g. in an amount effective in combination with the zinc phosphate to reduce erosion, dentinal hypersensitivity and/or plaque accumulation, for example in an amount of about 1-10% of the total composition weight in the case of a dentifrice, or for example about 0.5-2 wt. %, e.g., about 1% in the case of a mouthwash.
1.8. Any of the preceding compositions comprising a basic amino acid, e.g., arginine, in an amount sufficient to raise the pH of the formulation to greater than pH 8, e.g., to pH 8.5-10.
1.9. Any of the preceding compositions further comprising additional zinc ion sources, e.g., selected from zinc citrate, zinc sulfate, zinc silicate, zinc lactate, zinc oxide, and combinations thereof; for example in one embodiment, a dentifrice comprising 1% zinc phosphate and 1% zinc citrate.
1.10. Any of the preceding compositions comprising an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these, e.g., in an amount of 1-20%, e.g., 2-8%, e.g., ca. 5%, by weight of the composition.
1.11. Any of the foregoing compositions comprising buffering agents, e.g., sodium phosphate buffer (e.g., sodium phosphate monobasic and disodium phosphate).
1.12. Any of the foregoing compositions comprising a humectant, e.g., selected from glycerin, sorbitol, propylene glycol, polyethylene glycol, xylitol, and mixtures thereof, e.g. comprising at least 20%, e.g., 20-40%, e.g., 25-35% glycerin.
1.13. Any of the preceding compositions comprising one or more surfactants, e.g., selected from anionic, cationic, zwitterionic, and nonionic surfactants, and mixtures thereof, e.g., comprising an anionic surfactant, e.g., a surfactant selected from sodium lauryl sulfate, sodium ether lauryl sulfate, and mixtures thereof, e.g. in an amount of from about 0.3% to about 4.5% by weight, e.g. 1-2% sodium lauryl sulfate (SLS); and/or a zwitterionic surfactant, for example a betaine surfactant, for example cocamidopropylbetaine, e.g. in an amount of from about 0.1% to about 4.5% by weight, e.g. 0.5-2% cocamidopropylbetaine.
1.14. Any of the preceding compositions further comprising a viscosity modifying amount of one or more of polysaccharide gums, for example xanthan gum or carrageenan, silica thickener, and combinations thereof.
1.15. Any of the preceding compositions comprising gum strips or fragments.
1.16. Any of the preceding compositions further comprising flavoring, fragrance and/or coloring.
1.17. Any of the foregoing compositions comprising an effective amount of one or more antibacterial agents, for example comprising an antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing; e.g., comprising triclosan or cetylpyridinium chloride.
1.18. Any of the foregoing compositions comprising an antibacterially effective amount of triclosan, e.g. 0.1 -0.5%, e.g. about 0.3%.
1.19. Any of the preceding compositions further comprising a whitening agent, e.g., a selected from the group consisting of peroxides, metal chlorites, perborates, percarbonates, peroxyacids, hypochlorites, and combinations thereof.
1.20. Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate);
1.21. Any of the preceding compositions further comprising an agent that interferes with or prevents bacterial attachment, e.g., solbrol or chitosan.
1.22. Any of the preceding compositions further comprising a source of calcium and phosphate selected from (i) calcium-glass complexes, e.g., calcium sodium phosphosilicates, and (ii) calcium-protein complexes, e.g., casein phosphopeptide-amorphous calcium phosphate
1.23. Any of the preceding compositions further comprising a soluble calcium salt, e.g., selected from calcium sulfate, calcium chloride, calcium nitrate, calcium acetate, calcium lactate, and combinations thereof.
1.24. Any of the preceding compositions further comprising a physiologically or orally acceptable potassium salt, e.g., potassium nitrate or potassium chloride, in an amount effective to reduce dentinal sensitivity.
1.25. Any of the foregoing compositions further comprising an anionic polymer, e.g., a synthetic anionic polymeric polycarboxylate, e.g., wherein the anionic polymer is selected from 1:4 to 4:1 copolymers of maleic anhydride or acid with another polymerizable ethylenically unsaturated monomer; e.g., wherein the anionic polymer is a methyl vinyl ether/maleic anhydride (PVM/MA) copolymer having an average molecular weight (M.W.) of about 30,000 to about 1,000,000, e.g. about 300,000 to about 800,000, e.g., wherein the anionic polymer is about 1-5%, e.g., about 2%, of the weight of the composition.
1.26. Any of the preceding compositions further comprising a breath freshener, fragrance or flavoring.
1.27. Any of the foregoing compositions, wherein the pH of the composition is either acidic or basic, e.g., from pH 4 to pH 5.5 or from pH 8 to pH 10.
1.28. Any of the foregoing compositions which is a dentifrice, wherein the composition comprises
   1-3%, e.g., ca. 2% zinc phosphate;
   2-8%, e.g., ca. 5% L-arginine (free base);
   2-8%, e.g., ca. 5% alkali phosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these.
   700-2000 ppm, e.g., ca. 1450ppm fluoride, e.g., 0.3-0.4%, e.g., ca. 0.32% sodium fluoride;
   in a silica abrasive dentifrice base.
1.29. Any of the foregoing compositions which is a mouthwash, wherein the composition comprises
   5-10%, e.g., about 7.5% glycerin;
   3-7%, e.g., about 5.5% sorbitol;
   5-10%, e.g., about 7% propylene glycol;
   0-0.1% TSPP;
   0.01-1% sweetener, e.g, about 0.02% saccharin;
   0.01-1%, e.g., about 0.05% citric acid;
   0-0.1 % xanthan;
   0.005-0.05%, e.g., about 0.028% zinc phosphate;
   0 - 0.01% cetyl pyridinium chloride;
   0.01 - 0.1%, e.g., about 0.05%, potassium sorbate;
   0.05-1%, e.g., about 0.1-0.2% flavoring;
   0.1-2%, e.g., about 1% cocamidopropylbetaine;
   Water (optionally together with any additional ingredients) to make up balance, e.g., about 70-85%, e.g., about 80%, water.
1.30. Any of the foregoing compositions comprising substantially the same ingredients as in the test formulation in Example 1 or in Example 4 below.
1.31. Any of the preceding compositions effective upon application to the oral cavity, e.g., with brushing, to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; and/or (xv) promote systemic health, including cardiovascular health, e.g., by reducing potential for systemic infection via the oral tissues.
1.32. A composition obtained or obtainable by combining the ingredients as set forth in any of the preceding compositions.

The disclosure further provides the use of zinc phosphate in the manufacture of an oral care composition, e.g., a dentifrice, and in methods for enhancing the level of zinc in the enamel. In some embodiments, the zinc phosphate is added to the dentifrice as a preformed salt.

The disclosure further provides methods of using the compositions of the invention, to increase zinc levels in the enamel and to treat, reduce or control the incidence of enamel erosion, comprising applying a composition as described above, e.g., any of Composition 1, et seq., to the teeth, e.g., by brushing. In various embodiments, the invention provides to (i) reduce hypersensitivity of the teeth, (ii) to reduce plaque accumulation, (iii) reduce or inhibit demineralization and promote remineralization of the teeth, (iv) inhibit microbial biofilm formation in the oral cavity, (v) reduce or inhibit gingivitis, (vi) promote healing of sores or cuts in the mouth, (vii) reduce levels of acid producing bacteria, (viii) to increase relative levels of non-cariogenic and/or non-plaque forming bacteria, (ix) reduce or inhibit formation of dental caries, (x), reduce, repair or inhibit pre-carious lesions of the enamel, e.g., as detected by quantitative light-induced fluorescence (QLF) or electrical caries measurement (ECM), (xi) treat, relieve or reduce dry mouth, (xii) clean the teeth and oral cavity, (xiii) reduce erosion, (xiv) whiten teeth; (xv) reduce tartar build-up, and/or (xvi) promote systemic health, including cardiovascular health, e.g., by reducing potential for systemic infection via the oral tissues, comprising applying any of Compositions 1, et seq. as described above to the oral cavity of a person in need thereof, e.g., by brushing the teeth one or more times per day with any of Compositions 1, et seq. The invention further provides Compositions 1, et seq. for use in any of these methods.

*Active Agents*: The compositions of the invention may comprise various agents which are active to protect and enhance the strength and integrity of the enamel and tooth structure and/or to reduce bacteria and associated tooth decay and/or gum disease. Effective concentration of the active ingredients used herein will depend on the particular agent and the delivery system used. It is understood that a toothpaste for example will typically be diluted with water upon use, while a mouth rinse typically will not be. Thus, an effective concentration of active in a toothpaste will ordinarily be 5-15x higher than required for a mouth rinse. The concentration will also depend on the exact salt or polymer selected. For example, where the active agent is provided in salt form, the counterion will affect the weight of the salt, so that if the counterion is heavier, more salt by weight will be required to provide the same concentration of active ion in the final product. Arginine, where present, may be present at levels from, e.g., about 0.1 to about 20 wt %(expressed as weight of free base), e.g., about 1 to about 10 wt % for a consumer toothpaste or about 7 to about 20 wt % for a professional or prescription treatment product. Fluoride where present may be present at levels of, e.g., about 25 to about 25,000 ppm, for example about 750 to about 2,000 ppm for a consumer toothpaste, or about 2,000 to about 25,000 ppm for a professional or prescription treatment product. Levels of antibacterial agents will vary similarly, with levels used in toothpaste being e.g., about 5 to about 15 times greater than used in mouthrinse. For example, a triclosan toothpaste may contain about 0.3 wt % triclosan.

*Fluoride Ion Source*: The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al. Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. In certain embodiments, the oral care composition of the invention may also contain a source of fluoride ions or fluorine-providing ingredient in amounts sufficient to supply about 25 ppm to about 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application. A toothpaste for general consumer use would typically have about 1000 to about 1500 ppm, with pediatric toothpaste having somewhat less. A dentifrice or coating for professional application could have as much as about 5,000 or even about 25,000 ppm fluoride. Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counterion in the salt.

*Amino acids*: In some embodiments, the compositions of the invention comprise an amino acid. In particular embodiments, the amino acid may be a basic amino acid. By "basic amino acid" is meant the naturally occurring basic amino acids, such as arginine, lysine, and histidine, as well as any basic amino acid having a carboxyl group and an amino group in the molecule, which is water-soluble and provides an aqueous solution with a pH of about 7 or greater. Accordingly, basic amino acids include, but are not limited to, arginine, lysine, citrulline, ornithine, creatine, histidine, diaminobutanoic acid, diaminoproprionic acid, salts thereof or combinations thereof. In a particular embodiment, the basic amino acids are selected from arginine, citrulline, and ornithine. In certain embodiments, the basic amino acid is arginine, for example, 1-arginine, or a salt thereof. In other embodiments, the amino acid is quaternized, i.e., the amino group is additionally substituted to form a quaternary ammonium moiety, which may form an inner salt with the carboxyl group, for example, betaine (*N,N,N*-trimethylglycine).

In various embodiments, the amino acid is present in an amount of about 0.5 wt. % to about 20 wt. % of the total composition weight, about 0.5 wt. % to about 10 wt. % of the total composition weight, for example about 1.5 wt. %, about 3.75 wt. %, about 5 wt. %, or about 7.5 wt. % of the total composition weight in the case of a dentifrice, or for example about 0.5-2 wt. %, e.g., about 1% in the case of a mouthwash.

*Abrasive*: The compositions of the invention, e.g. Composition 1 et seq. include silica abrasives, and may comprise additional abrasives, e.g., a calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, or combinations thereof.

Other silica abrasive polishing materials useful herein, as well as the other abrasives, generally have an average particle size ranging between about 0.1 and about 30 microns, about between 5 and about 15 microns. The silica abrasives can be from precipitated silica or silica gels, such as the silica xerogels described in U.S. Pat. No. 3,538,230, to Pader et al. and U.S. Pat. No. 3,862,307, to Digiulio. Particular silica xerogels are marketed under the trade name Syloid® by the W. R. Grace & Co., Davison Chemical Division. The precipitated silica materials include those marketed by the J. M. Huber Corp. under the trade name Zeodent®, including the silica carrying the designation Zeodent 115 and 119. These silica abrasives are described in U.S. Pat. No. 4,340,583, to Wason. In certain embodiments, abrasive materials useful in the practice of the oral care compositions in accordance with the invention include silica gels and precipitated amorphous silica having an oil absorption value of less than about 100 cc/100 g silica and in the range of about 45 cc/100 g to about 70 cc/100 g silica. Oil absorption values are measured using the ASTA Rub-Out Method D281. In certain embodiments, the silicas are colloidal particles having an average particle size of about 3 microns to about 12 microns, and about 5 to about 10 microns. Low oil absorption silica abrasives particularly useful in the practice of the invention are marketed under the trade designation Sylodent XWA® by Davison Chemical Division of W.R. Grace & Co., Baltimore, Md. 21203. Sylodent 650 XWA®, a silica hydrogel composed of particles of colloidal silica having a water content of 29% by weight averaging about 7 to about 10 microns in diameter, and an oil absorption of less than about 70 cc/100 g of silica is an example of a low oil absorption silica abrasive useful in the practice of the present invention.

*Foaming agents:* The oral care compositions of the invention also may include an agent to increase the amount of foam that is produced when the oral cavity is brushed. Illustrative examples of agents that increase the amount of foam include, but are not limited to polyoxyethylene and certain polymers including, but not limited to, alginate polymers. The polyoxyethylene may increase the amount of foam and the thickness of the foam generated by the oral care carrier component of the present invention. Polyoxyethylene is also commonly known as polyethylene glycol ("PEG") or polyethylene oxide. The polyoxyethylenes suitable for this invention will have a molecular weight of about 200,000 to about 7,000,000. In one embodiment the molecular weight will be about 600,000 to about 2,000,000 and in another embodiment about 800,000 to about 1,000,000. Polyox® is the trade name for the high molecular weight polyoxyethylene produced by Union Carbide. The polyoxyethylene may be present in an amount of about 1% to about 90%, in one embodiment about 5% to about 50% and in another embodiment about 10% to about 20% by weight of the oral care carrier component of the oral care compositions of the present invention. Where present, the amount of of foaming agent in the oral care composition (i.e., a single dose) is about 0.01 to about 0.9 % by weight, about 0.05 to about 0.5% by weight, and in another embodiment about 0.1 to about 0.2 % by weight.

*Surfactants*: The compositions useful in the invention may contain anionic surfactants, for example:
i. water-soluble salts of higher fatty acid monoglyceride monosulfates, such as the sodium salt of the monosulfated monoglyceride of hydrogenated coconut oil fatty acids such as sodium N-methyl N-cocoyl taurate, sodium cocomonoglyceride sulfate,
ii. higher alkyl sulfates, such as sodium lauryl sulfate,
iii. higher alkyl-ether sulfates, e.g., of formula CH₃(CH₂)ₘCH₂(OCH₂CH₂)ₙOSO₃X, wherein m is 6-16, e.g., 10, n is 1-6, e.g., 2, 3 or 4, and X is Na or K, for example sodium laureth-2 sulfate (CH₃(CH₂)₁₀CH₂(OCH₂CH₂)₂OSO₃Na).
iv. higher alkyl aryl sulfonates such as sodium dodecyl benzene sulfonate (sodium lauryl benzene sulfonate)
v. higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate.

By "higher alkyl" is meant, e.g., C₆₋₃₀ alkyl. In particular embodiments, the anionic surfactant is selected from sodium lauryl sulfate and sodium ether lauryl sulfate. The anionic surfactant may be present in an amount which is effective, e.g., > 0.01 % by weight of the formulation, but not at a concentration which would be irritating to the oral tissue, e.g., <10%, and optimal concentrations depend on the particular formulation and the particular surfactant. For example, concentrations used or a mouthwash are typically on the order of one tenth that used for a toothpaste. In one embodiment, the anionic surfactant is present in a toothpaste at from about 0.3% to about 4.5% by weight, e.g., about 1.5%. The compositions of the invention may optionally contain mixtures of surfactants, e.g., comprising anionic surfactants and other surfactants that may be anionic, cationic, zwitterionic or nonionic. Generally, surfactants are those which are reasonably stable throughout a wide pH range. Surfactants are described more fully, for example, in U.S. Pat. No. 3,959,458, to Agricola et al.; U.S. Pat. No. 3,937,807, to Haefele; and U.S. Pat. No. 4,051,234, to Gieske et al. In certain embodiments, the anionic surfactants useful herein include the water-soluble salts of alkyl sulfates having about 10 to about 18 carbon atoms in the alkyl radical and the water-soluble salts of sulfonated monoglycerides of fatty acids having about 10 to about 18 carbon atoms. Sodium lauryl sulfate, sodium lauroyl sarcosinate and sodium coconut monoglyceride sulfonates are examples of anionic surfactants of this type. In a particular embodiment, the composition of the invention, e.g., Composition 1, et seq., comprises sodium lauryl sulfate.

The surfactant or mixtures of compatible surfactants can be present in the compositions of the present invention in about 0.1% to about 5.0%, in another embodiment about 0.3% to about 3.0% and in another embodiment about 0.5% to about 2.0% by weight of the total composition.

*Tartar control agents:* In various embodiments of the present invention, the compositions comprise an anticalculus (tartar control) agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. The invention thus may comprise phosphate salts in addition to the zinc phosphate. In particular embodiments, these salts are alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1% of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

*Flavoring Agent*: The oral care compositions of the invention may also include a flavoring agent. Flavoring agents which are used in the practice of the present invention include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint. The flavoring agent may be incorporated in the oral composition at a concentration of about 0.1 to about 5% by weight e.g. about 0.5 to about 1.5% by weight.

*Polymers*: The oral care compositions of the invention may also include additional polymers to adjust the viscosity of the formulation or enhance the solubility of other ingredients. Such additional polymers include polyethylene glycols, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts.

Silica thickeners, which form polymeric structures or gels in aqueous media, may be present. Note that these silica thickeners are physically and functionally distinct from the particulate silica abrasives also present in the compositions, as the silica thickeners are very finely divided and provide little or no abrasive action. Other thickening agents are carboxyvinyl polymers, carrageenan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate can also be used as component of the thickening composition to further improve the composition's texture. In certain embodiments, thickening agents in an amount of about 0.5% to about 5.0% by weight of the total composition are used.

The compositions of the invention may include an anionic polymer, for example in an amount of from about 0.05 to about 5%. Such agents are known generally for use in dentifrice, although not for this particular application, useful in the present invention are disclosed in U.S. Pat. Nos. 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of about 30,000 to about 1,000,000, most preferably about 300,000 to about 800,000. These copolymers are available for example as Gantrez. e.g., AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from about 0.05 to about 3% by weight. Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility. A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid. Another useful class of polymeric agents includes polyamino acids containing proportions of anionic surface-active amino acids such as aspartic acid, glutamic acid and phosphoserine, e.g. as disclosed in U.S. Pat. No. 4,866,161 Sikes et al.

*Water:* The oral compositions may comprise significant levels of water. Water employed in the preparation of commercial oral compositions should be deionized and free of organic impurities. The amount of water in the compositions includes the free water which is added plus that amount which is introduced with other materials.

*Humectants:* Within certain embodiments of the oral compositions, it is also desirable to incorporate a humectant to prevent the composition from hardening upon exposure to air. Certain humectants can also impart desirable sweetness or flavor to dentifrice compositions. Suitable humectants include edible polyhydric alcohols such as glycerine, sorbitol, xylitol, propylene glycol as well as other polyols and mixtures of these humectants. In one embodiment of the invention, the principal humectant is glycerin, which may be present at levels of greater than 25%, e.g. 25-35% about 30%, with 5% or less of other humectants.

*Other optional ingredients:* In addition to the above-described components, the embodiments of this invention can contain a variety of optional dentifrice ingredients some of which are described below. Optional ingredients include, for example, but are not limited to, adhesives, sudsing agents, flavoring agents, sweetening agents, additional antiplaque agents, abrasives, and coloring agents. These and other optional components are further described in U.S. Pat. No. 5,004,597, to Majeti; U.S. Pat. No. 3,959,458 to Agricola et al. and U.S. Pat. No. 3,937,807, to Haefele.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

### EXAMPLES

### Example 1

Test dentifrice comprising 2% zinc phosphate in combination with 5% arginine, 1450 ppm fluoride, and phosphates is prepared. This dentifrice has a pH of 9.13, consistent with the relatively high arginine content. Dentifrices comprising 2% zinc citrate, 1% zinc citrate, 1% zinc citrate + 1% zinc phosphate, no zinc, or no arginine are also prepared in accordance with the following formulations (ingredients by weight of composition):

**Table 1**

| Ingredient | 2% Zinc Phosphate | 2% Zinc Citrate | 1% Zinc Citrate | 1% Zinc Phosphate + 1% Zinc Citrate | No Arginine |
|---|---|---|---|---|---|
| PEG600 | 3.0 | 3.0 | 3.0 | 3.0 | 3.0 |
| CMC-7 | 0.65 | 0.65 | 0.65 | 0.65 | 0.65 |
| Xanthan | 0.2 | 0.2 | 0.2 | 0.2 | 0.2 |
| Sorbitol | 27.0 | 27.4 | 28.4 | 28.4 | 28.4 |
| Glycerin | 20.0 | 20.0 | 20.0 | 20.0 | 20.0 |
| Saccharin | 0.3 | 0.3 | 0.3 | 0.3 | 0.3 |
| Tetrasodium pyrophosphate | 0.5 | 0.5 | 0.5 | 0.5 | 0.5 |
| Calcium pyrophosphate | 0.25 | 0.25 | 0.25 | 0.25 | 0.25 |
| Sodium phosphate dibasic | 3.5 | 3.5 | 3.5 | 3.5 | 3.5 |
| Sodium fluoride (to provide 1450 ppm fluoride) | 0.32 | 0.32 | 0.32 | 0.32 | 0.32 |
| Water | QS | QS | QS | QS | QS |
| Titanium dioxide | 0.5 | 0.5 | 0.5 | 0.5 | 0.5 |
| Abrasive silica | 8.0 | 8.0 | 8.0 | 8.0 | 8.0 |
| Thickener silica | 8.0 | 8.0 | 8.0 | 8.0 | 8.0 |
| L-Arginine | 5.0 | 5.0 | 5.0 | 5.0 | ------ |
| Sodium lauryl sulfate | 1.5 | 1.5 | 1.5 | 1.5 | 1.5 |
| Flavoring | 1.2 | 1.2 | 1.2 | 1.2 | 1.2 |
| Zinc Phosphate | 2.0 | ------ | ------ | 1.0 | ------ |
| Zinc Citrate | ------ | 2.0 | 1.0 | 1.0 | 1.0 |

### Example 2

Dentifrice of Example 1 containing 2% zinc phosphate, together with arginine and fluoride, shows superior efficacy towards acid challenge compared to control dentifrice comprising 1% zinc citrate.

An *in vitro* methodology is used to determine the enamel protection activity of the formulation prototypes of Example 1. Enamel substrates (N = 6/8 per cell) are prepared by embedding bovine incisors in methacrylate-based resin and polishing with 600 and 1200 grit carbide paper consecutively. Care is taken not to penetrate the dentin layer while polishing the enamel to a mirror finish. Prior to testing, all enamel substrates are pre-etched with 5% citric acid for 30 sec. Half the side of each substrate is masked with acid resistant tape to protect the surface as control surface. The model used to evaluate the products alternated 1-min product treatment periods with 2-min acid exposure periods according to the daily sequence of T - C - C - C - C - T (T = product treatment, C = acid challenge). The acid challenge is performed with a 1% aqueous solution of citric acid (unbuffered) adjusted to pH = 3.8 with NaOH. All enamel substrates are kept in a sterile artificial saliva solution at 37 °C while not undergoing treatment or challenge. This regimen is conducted for a total of five days, at the end of which a microhardness analysis is used to quantify the amount of enamel lost due to erosion on each enamel substrate on protected and exposed surface. The change in percentage hardness is calculated. Without treatment, using deionized water in place of test dentifrice, the change in percentage hardness is very high, ca. 80%, with slight variation from experiment to experiment depending on the particular substrate.

The formula containing 2% zinc phosphate is effective against demineralization in this *in vitro* pH-cycling model designed to investigate the protective effect of treatments on early enamel dissolution, with a reduction in hardness following repeated acid challenge of only 24.3%, which is less than the 26.3% reduction in hardness seen for the 1% zinc citrate formulation. A separate experiment shows that increasing the amount of zinc citrate in the formulation from 1% to 2% does not increase efficacy against acid challenge. The maximal effect is already achieved at 1%. Thus the effectiveness of a zinc phosphate formulation in this assay is superior to, or at least as good as, the maximum effect achievable using a zinc citrate formulation, which is surprising in view of the belief in the art that zinc phosphate is relatively inert in the oral cavity.

Further experiments are conducted to evaluate formulations comprising zinc phosphate (ZnP) with and without arginine, and formulations comprising 1% zinc phosphate and zinc citrate. A commercial enamel-protecting toothpaste (Commercial 1) comprising sodium fluoride and potassium nitrate is included as a positive control. (NB: Preparations made from different human teeth are used for each set of experiments, so there may be some variation between sets of experiments):

**Table 2**

| | No treatment | ZnP | ZnP + Zn Citrate | ZnP + Arg | Commercial 1 |
|---|---|---|---|---|---|
| % Reduction in hardness | 67.56 | 34.13 | 44.19 | 31.26 | 32.96 |

In this series of experiments, the zinc phosphate with arginine is at least as good as the positive control, and unexpectedly, is significantly better than the combination of zinc phosphate and zinc citrate.

### Example 3

Electron spectroscopy for chemical analysis (ESCA, also known as x-ray photoelectron spectroscopy or XPS) is a surface analysis technique used for obtaining chemical information about the surfaces of solid materials. The materials characterization method utilizes an x-ray beam to excite a solid sample resulting in the emission of photoelectrons. An energy analysis of these photoelectrons provides both elemental and chemical bonding information about a sample surface. The relatively low kinetic energy of the photoelectrons gives ESCA a sampling depth of approximately 30 Å. ESCA is used to analyze the mineral content of the enamel before and after use of test vs. control dentifrices on polished and etched enamel substrates prepared and treated as described in the foregoing examples, confirming that zinc is delivered to the enamel surface and that zinc remains on the surface even after acid challenges, as well as confirming effective delivery of fluoride.

The ESCA data for the enamel samples treated with the various Zn / arginine / phosphate anti-erosion pastes of Example 1 are shown in Table 3 (below), setting forth the elements detected and their respective atomic concentrations. All samples are analyzed after each polishing, etching and treatment step. For simplicity, only the mean composition for all samples is presented for the polished and etched surfaces. In all cases, three to four separate areas are analyzed for each sample. For the treated samples, the compositions of the individual areas, as well as the mean composition for each sample are presented.

C and N are detected on the polished and etched samples from surface organics. Ca and P are detected from the hydroxyapatite (HAP) in the enamel, with P/Ca ratios typical for enamel surfaces. The mean P/Ca ratio for the etched surfaces is slightly lower than that for the polished surfaces, indicating a reduction in surface phosphate relative to Ca occurs after etching. A low level of Zn is detected for the polished surfaces, which is removed by the etching. Fluoride is also detected for both the polished and etched surfaces at low levels. A low level of Na is also observed. Overall, the compositions of the polished and etched enamel surfaces are typical for bovine enamel.

C and N are detected on the surfaces of all the treated samples from surface organics. The C levels for all samples are higher than that for the etched surfaces, due to the presence of residual organics, most likely from the pastes. The N levels are also higher for the treated samples relative to the etched surfaces.

Ca and P are also detected on the surfaces of the samples, from the HAP in the enamel. The P/Ca ratios for the treated samples are higher than that for the etched surfaces, indicating an excess of phosphate relative to Ca. For all the samples, the Na concentrations are also significantly greater than that for the etched enamel. The higher phosphate and Na levels on the treated samples suggests that Na phosphate from the paste has been deposited on the enamel surfaces. Si is also detected in low amounts on the treated enamel, due to residual silica from the pastes.

For the enamel samples treated with the pastes containing either zinc citrate or zinc phosphate, elevated zinc levels are observed on the surface. As for the previous studies, the Zn/Ca ratios provide the best means for comparison of Zinc uptake on the enamel surfaces. The Zn/Ca ratio for enamel treated with the Zinc phosphate paste is greater than those for the Zinc citrate pastes. This indicates that the zinc phosphate paste deposits greater zinc on the enamel than the zinc citrate products. On a molar basis, zinc phosphate contains greater zinc than an equal weight of zinc citrate. This may account for the greater zinc detected on the enamel treated with the zinc phosphate paste, but it is any event clear that zinc phosphate is effective in delivering zinc to the enamel surface. Overall, the Zn/Ca ratios observed for the samples in the present study are about double those for the enamel samples of the pH cycling study reported previously.

The ESCA zinc peak positions can provide information on the chemical state of zinc on the enamel surfaces. The zinc peak positions for all the samples treated with Zinc containing pastes are the same. This suggests that the zinc present on all the samples is in a similar chemical form. In addition, the zinc peak positions for the zinc paste treated samples are the same as those for the background zinc on the polished enamel surfaces. The background zinc inherently present in enamel is probably in the form of a zinc HAP. Thus the data suggest that the Zinc on the treated enamel may also be in the form of a zinc HAP.

F in the form of fluoride is detected on the surfaces of all the paste treated enamel samples. Relative fluoride uptake between samples is also best determined using the F/Ca ratios. The data indicate that enamel treated with 1% and 2% Zinc citrate pastes had similar F/Ca ratios, suggesting similar F uptake. The enamel treated with the Zinc citrate w/o arginine and Zn₃(PO4)₂ pastes exhibited slightly lower F/Ca ratios than the other samples. The lower fluoride uptake for the latter two treatments however is within normal variation for this type of study.

The ESCA results indicate that zinc deposition occurs for enamel treated with pastes containing either zinc citrate or zinc phosphate. Zinc deposition is greater for the paste containing zinc phosphate compared to the zinc citrate pastes. The data also suggest that zinc may be present on the surface in the form of a zinc-HAP. Elevated levels of fluoride are also observed for all the paste treated samples.

The detailed results of the analysis are presented in Table 3 (below).

**Table 3**

| **ESCA Analysis of Enamel - Zn / Arginine / PO₄ / NaF Anti-Erosion Paste** | | | | | | | | | | | | | | | |
|---|---|---|---|---|---|---|---|---|---|---|---|---|---|---|---|
| **Sample** | | | | **Atomic Percent** | | | | | | | | | **Ratio** | | |
| | | | | **C** | **O** | **N** | **Ca** | **P** | **Zn** | **Na** | **F** | **Si** | **P/Ca** | **Zn/Ca** | **F/Ca** |
| **Polished** - **all 5 samples** | | | | | | | | | | | | | | | |
| | | ***mean*** | | **23.84** | **49.56** | **0.58** | **14.39** | **11.04** | **0.09** | **0.42** | **0.10** | **-** | **0.77** | **0.007** | **0.007** |

| **Etched - all 5 samples** | | | | | | | | | | | | | | | |
|---|---|---|---|---|---|---|---|---|---|---|---|---|---|---|---|
| | | ***mean*** | | **31.88** | **44.05** | **0.46** | **13.22** | **9.75** | **0.00** | **0.51** | **0.13** | **-** | **0.74** | **0.000** | **0.010** |
| | | | | | | | | | | | | | | | |

| **1% Zn citrate w/o arginine** | | | | | | | | | | | | | | | |
|---|---|---|---|---|---|---|---|---|---|---|---|---|---|---|---|
| *treated* | | | | 40.57 | 36.10 | 0.82 | 8.78 | 8.32 | 0.54 | 3.35 | 0.48 | 1.05 | 0.95 | 0.062 | 0.055 |
| | | | | 43.35 | 34.21 | 0.99 | 8.49 | 7.94 | 0.55 | 3.08 | 0.50 | 0.88 | 0.94 | 0.065 | 0.059 |
| | | | | 44.50 | 33.64 | 1.00 | 8.47 | 7.82 | 0.53 | 2.88 | 0.49 | 0.67 | 0.92 | 0.063 | 0.058 |
| | | ***mean*** | | ***42.81*** | ***34.65*** | ***0.94*** | ***8.58*** | ***8.03*** | ***0.54*** | ***3.10*** | ***0.49*** | ***0.87*** | ***0.94*** | ***0*.*063*** | ***0***.***057*** |
| | | | | | | | | | | | | | | | |

| **1% Zn citrate w/ arginine** | | | | | | | | | | | | | | | |
|---|---|---|---|---|---|---|---|---|---|---|---|---|---|---|---|
| *treated* | | | | 43.06 | 37.74 | 0.93 | 6.23 | 6.11 | 0.33 | 2.88 | 0.45 | 2.27 | 0.98 | 0.053 | 0.072 |
| | | | | 42.48 | 36.77 | 0.74 | 7.25 | 6.84 | 0.45 | 2.50 | 0.51 | 2.43 | 0.94 | 0.062 | 0.070 |
| | | * | | 38.71 | 37.10 | 0.82 | 8.14 | 8.00 | 0.47 | 3.99 | 0.51 | 1.15 | 0.98 | 0.058 | 0.063 |
| | | ***mean*** | | ***41.42*** | ***37.20*** | ***0.83*** | ***7.21*** | ***6.98*** | ***0.42*** | ***3.12*** | ***0.49*** | ***1.95*** | ***0.97*** | ***0.058*** | ***0.068*** |
| | | | | | | | | | | | | | | | |

| **2% Zn citrate w/ arginine** | | | | | | | | | | | | | | | |
|---|---|---|---|---|---|---|---|---|---|---|---|---|---|---|---|
| *treated* | | * | | 47.28 | 31.68 | 1.19 | 7.15 | 7.42 | 0.39 | 2.42 | 0.49 | 1.15 | 1.04 | 0.055 | 0.069 |
| | | | | 42.77 | 34.84 | 1.21 | 8.22 | 8.43 | 0.55 | 2.60 | 0.55 | 0.83 | 1.03 | 0.067 | 0.067 |
| | | | | 43.07 | 34.28 | 1.26 | 7.94 | 8.22 | 0.51 | 3.12 | 0.52 | 1.08 | 1.04 | 0.064 | 0.065 |
| | | ***mean*** | | ***44***.***37*** | ***33.60*** | ***1.2**2* | *7.77* | ***8.02*** | ***0.48*** | ***2.71*** | ***0.52*** | ***1.02*** | ***1.03*** | ***0.062*** | ***0.067*** |
| | | | | | | | | | | | | | | | |

| **2% Zn₃(PO4)₂ w/ arginine** | | | | | | | | | | | | | | | |
|---|---|---|---|---|---|---|---|---|---|---|---|---|---|---|---|
| *treated* | | ***** | | 43.90 | 34.75 | 1.11 | 6.21 | 6.63 | 0.47 | 4.11 | 0.31 | 1.58 | 1.07 | 0.076 | 0.050 |
| | | | | 44.59 | 34.50 | 1.07 | 6.79 | 6.81 | 0.54 | 2.52 | 0.36 | 2.82 | 1.00 | 0.080 | 0.053 |
| | | | | 37.08 | 40.09 | 0.91 | 7.86 | 7.62 | 0.72 | 2.80 | 0.43 | 2.49 | 0.97 | 0.092 | 0.055 |
| | | | | 39.47 | 37.94 | 1.09 | 7.48 | 7.89 | 0.59 | 3.67 | 0.42 | 1.44 | 1.05 | 0.079 | 0.056 |
| | | ***mean*** | | ***41.26*** | ***36.82*** | ***1.05*** | ***7.09*** | ***7.24*** | ***0.58*** | ***3.28*** | ***0.38*** | ***2.08*** | ***1.01*** | ***0*.*082*** | ***0*.*053*** |
| | | * - Cl also detected | | | | | | | | | | | | | |

As this data shows, the delivery of zinc to the enamel using the zinc phosphate formulation (mean 0.58) is actually higher than the delivery of zinc using any of the zinc citrate formulations. The incorporation of zinc into the enamel, possibly through formation of a zinc-hydroxyapatite material, is advantageous in view of the beneficial effects of zinc in controlling erosion and reducing bacterial biofilm deposition. Fluoride delivery with the zinc phosphate formulation is also in an acceptable range, showing significant deposition of fluoride compared to the control, similar to the formulation comprising zinc citrate without arginine.

### Example 4

Although zinc phosphate is insoluble in water, it is found to be soluble in acidic solutions. Formulations comprising e.g., 2% lactic acid (ca. pH 2.4), or even as little as 0.05% citric acid (ca. pH 4), with zinc phosphate at 0.01-0.03% do not exhibit precipitation. It is found that the solubility of zinc phosphate can be enhanced in a formulation comprising an amino acid and/or a betaine surfactant. A stable mouthwash formulation is provided as follows:

| **INGREDIENT** | **WEIGHT %** |
|---|---|
| Sorbitol | 5.5 |
| Glycerin | 7.5 |
| Propylene glycol | 7 |
| Sodium saccharin | 0.02 |
| Citric acid (anhydrous) | 0.05 |
| Zinc phosphate | 0.028 |
| Flavor /dye | 0.12 |
| Potassium sorbate | 0.05 |
| Cocamidopropyl betaine | 1 |
| Water | QS |
| **TOTAL** | **100** |

## Claims

1. A dentifrice comprising (i) zinc phosphate, and (ii) arginine in free or orally acceptable salt form in an amount of 0.5 to 10 % by weight, wherein the zinc phosphate is added to the dentifrice as a preformed salt in an amount of 0.5 to 4 % by weight.

2. The dentifrice of claim 1 wherein the dentifrice base comprises an abrasive.

3. The dentifrice of any of the foregoing claims comprising one or more humectants, and one or more surfactants.

4. The dentifrice of any of the foregoing claims further comprising an effective amount of: a fluoride ion source; one or more alkali phosphate salts; one or more antibacterial agents; or a combination of any two or more thereof.

5. The dentifrice of any of the foregoing claims further comprising: (i) a whitening agent; (ii) one or more sources of zinc ion in addition to the zinc phosphate; or a combination thereof.

6. The dentifrice of any of the foregoing claims comprising
1 to 3% zinc phosphate;
1 to 8% arginine;
2 to 8% alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and mixtures of any two or more of these;
700 to 2000 ppm fluoride;
in a silica abrasive dentifrice base.

7. A mouthwash comprising zinc phosphate and a basic amino acid in free or orally acceptable salt form, wherein the zinc phosphate is added to the mouthwash as a preformed salt, and wherein
the basic amino acid is arginine in an amount of 0.5 to 2 % by weight and the zinc phosphate is added in an amount of 0.005 to 0.05 % by weight.

8. The mouthwash of claim 7 further comprising an effective amount of: a fluoride ion source; one or more alkali phosphate salts; one or more antibacterial agents; or a combination of any two or more thereof.

9. The mouthwash of any one of claims 7 to 8, further comprising: (i) a whitening agent; (ii) one or more sources of zinc ion in addition to the zinc phosphate; or a combination thereof.

10. The mouthwash of any one of claims 7 to 9, comprising
0.1 to 2% zinc phosphate;
0.1 to 3% arginine;
2 to 8% alkali phosphate salts selected from sodium phosphate dibasic, potassium phosphate dibasic, dicalcium phosphate dihydrate, tetrasodium pyrophosphate, tetrapotassium pyrophosphate, calcium pyrophosphate, sodium tripolyphosphate, and mixtures of any two or more of these;
700 to 2000 ppm fluoride.

11. The mouthwash of any one of claims 7 to 9, wherein the composition comprises
5 to 10% glycerin;
3 to 7% sorbitol;
5 to 10% propylene glycol;
0 to 0.1% TSPP;
0.01 to 1% sweetener;
0.01 to 1% citric acid;
0 to 0.1 % xanthan;
0.005 to 0.05% zinc phosphate;
0 to 0.01% cetyl pyridinium chloride;
0.01 to 0.1% potassium sorbate;
0.05 to 1% flavoring; and
0.1 to 2% cocamidopropyl betaine.

12. The dentifrice of any one of claims 1 to 6 or the mouthwash of any one of claims 7 to 11, wherein the pH of the composition is acidic.

13. The dentifrice of any one of claims 1 to 6 or the mouthwash of any one of claims 7 to 11, wherein the pH of the composition is basic.

14. Use of zinc phosphate and arginine in free or orally acceptable salt form in the manufacture of a dentifrice or mouthwash, wherein the zinc phosphate is added to the dentifrice as a preformed salt in an amount of 0.5 to 4 % by weight, or wherein the zinc phosphate is added to the mouthwash as a preformed salt in an amount of 0.005 to 0.05% by weight, and wherein the arginine is present in the dentifrice or mouthwash in an amount of 0.5 to 10 % by weight.

15. A composition according to any of claims 1 to 13 for use in treating or reducing dental enamel erosion.

## Patentansprüche

1. Zahnreinigungsmittel, umfassend (i) Zinkphosphat und (ii) Arginin in freier oder oral annehmbarer Salzform in einer Menge von 0,5 bis 10 Gew.-%, worin das Zinkphosphat dem Zahnreinigungsmittel als ein vorgefertigtes Salz in einer Menge von 0,5 bis 4 Gew.-% zugegeben wird.

2. Zahnreinigungsmittel nach Anspruch 1, worin die Zahnreinigungsmittelbasis einen abrasiven Stoff umfasst.

3. Zahnreinigungsmittel nach irgendeinem der vorhergehenden Ansprüche, umfassend einen oder mehrere Befeuchtungsmittel und ein oder mehrere Tenside.

4. Zahnreinigungsmittel nach irgendeinem der vorhergehenden Ansprüche, weiterhin umfassend eine wirksame Menge aus: einer Fluoridionenquelle, einem oder mehreren Alkaliphosphatsalzen, einem oder mehreren antibakteriellen Mitteln, oder einer Kombination aus beliebigen zwei oder mehreren davon.

5. Zahnreinigungsmittel nach irgendeinem der vorhergehenden Ansprüche, weiterhin umfassend: (i) ein Aufhellungsmittel, (ii) eine oder mehrere Quellen für Zinkionen zusätzlich zu dem Zinkphosphat oder eine Kombination davon.

6. Zahnreinigungsmittel nach irgendeinem der vorhergehenden Ansprüche, umfassend:
1 bis 3 % Zinkphosphat;
1 bis 8 % Arginin;
2 bis 8 % Alkaliphosphatsalze, ausgewählt aus Natriumphosphat dibasisch, Kaliumphosphat dibasisch, Dicalciumphosphat-dihydrat, Tetranatriumpyrophosphat, Tetrakaliumpyrophosphat, Calciumpyrophosphat, Natriumtripolyphosphat, und Mischungen aus beliebigen zwei oder mehreren derselben,
700 bis 2000 ppm Fluoride;
in einer Silica-abrasiv-Zahreinigungsmittel-Basis.

7. Mundwasser umfassend Zinkphosphat und eine basische Aminosäure in freier oder oral annehmbarer Salzform, worin das Zinkphosphat dem Mundwasser als ein vorgefertigtes Salz zugegeben wird, und wobei die basische Aminosäure Arginin ist in einer Menge von 0,5 bis 2 Gew.-% und das Zinkphosphat wird in einer Menge von 0,0005 bis 0,05 Gew.-% zugegeben.

8. Mundwasser nach Anspruch 7, weiterhin umfassend eine wirksame Menge aus: einer Fluoridionenquelle, ein oder mehrere Alkaliphosphatsalze, ein oder mehrere antibakterielle Mittel oder eine Kombination aus beliebigen zwei oder mehreren davon.

9. Mundwasser nach irgendeinem der Ansprüche 7 bis 8, weiterhin umfassend: (i) ein Aufhellungsmittel, (ii) ein oder mehrere Quellen für Zinkionen zusätzlich zu dem Zinkphosphat oder eine Kombination davon.

10. Mundwasser nach irgendeinem der Ansprüche 7 bis 9, weiterhin umfassend:
0,1 bis 2 % Zinkphosphat,
0,1 bis 3 % Arginin,
2 bis 8 % Alkaliphosphatsalze ausgewählt aus Natriumphosphat dibasisch, Kaliumphosphat dibasisch, Dicalciumphosphat-dihydrat, Tetranatriumpyrophosphat, Tetrakaliumpyrophosphat, Calciumpyrophosphat, Natriumtripolyphosphat, und Mischungen aus beliebigen zwei oder mehreren derselben,
700 bis 2000 ppm Fluoride.

11. Mundwasser nach irgendeinem der Ansprüche 7 bis 9, worin die Zusammensetzung umfasst:
5 bis 10 % Glycerin,
3 bis 7 % Sorbitol,
5 bis 10 % Propylenglycol,
0 bis 0,1 % TSPP,
0,01 bis 1 % Süßungsmittel,
0,01 bis 1 % Zitronensäure,
0 bis 0,1 % Xanthan,
0,005 bis 0,05 % Zinkphosphat,
0 bis 0,01 % Cetylpyridiniumchlorid,
0,01 bis 0,1 % Kaliumsorbat,
0,05 bis 1 % Aromamittel, und
0,1 bis 2 % Cocamidopropylbetain.

12. Zahnreinigungsmittel nach irgendeinem der Ansprüche 1 bis 6 oder Mundwasser nach irgendeinem der Ansprüche 7 bis 11, worin der pH-Wert der Zusammensetzung sauer ist.

13. Zahnreinigungsmittel nach irgendeinem der Ansprüche 1 bis 6 oder Mundwasser nach irgendeinem der Ansprüche 7 bis 11, worin der pH-Wert der Zusammensetzung basisch ist.

14. Verwendung von Zinkphosphat und Arginin in freier oder oral annehmbarer Salzform in der Herstellung eines Zahnreinigungsmittels oder Mundwassers, worin das Zinkphosphat dem Zahnreinigungsmittel als ein vorgefertigtes Salz in einer Menge von 0,5 bis 4 Gew.-% zugegeben wird, oder worin das Zinkphosphat dem Mundwasser als ein vorgefertigtes Salz in einer Menge von 0,005 bis 0,05 Gew.-% zugegeben wird, und worin das Arginin in dem Zahnreinigungsmittel oder Mundwasser in einer Menge von 0,5 bis 10 Gew.-% vorliegt.

15. Zusammensetzung nach irgendeinem der Ansprüche 1 bis 13 zur Verwendung in der Behandlung oder Reduzierung von Zahnschmelzerosion.

## Revendications

1. Dentifrice comprenant (i) du phosphate de zinc, et (i) de l'arginine sous forme libre ou sous la forme d'un sel acceptable par voie orale en une quantité de 0,5 à 10 % en poids, dans lequel le phosphate de zinc est ajouté au dentifrice sous la forme d'un sel préformé en une quantité de 0,5 à 4 % en poids.

2. Dentifrice selon la revendication 1, dans lequel la base de dentifrice comprend un abrasif.

3. Dentifrice selon l'une quelconque des revendications précédentes, comprenant un ou plusieurs humectants, et un ou plusieurs tensioactifs.

4. Dentifrice selon l'une quelconque des revendications précédentes, comprenant en outre une quantité efficace de : une source d'ions fluorure ; un ou plusieurs sels de phosphate alcalin ; un ou plusieurs agents antibactériens ; ou une combinaison de deux ou plus de ceux-ci.

5. Dentifrice selon l'une quelconque des revendications précédentes, comprenant en outre : (i) un agent de blanchiment ; (ii) une ou plusieurs sources d'ion zinc en plus du phosphate de zinc ; ou une combinaison de ceux-ci.

6. Dentifrice selon l'une quelconque des revendications précédentes, comprenant
1 à 3 % de phosphate de zinc ;
1 à 8 % d'arginine ;
2 à 8 % de sels de phosphate alcalin choisis parmi le phosphate dibasique de sodium, le phosphate dibasique de potassium, l'hydrogénophosphate de calcium dihydrate, le pyrophosphate de tétrasodium, le pyrophosphate de tétrapotassium, le pyrophosphate de calcium, le tripolyphosphate de sodium et des mélanges de deux ou plus de ceux-ci ;
700 à 2 000 ppm de fluorure ;
dans une base de dentifrice abrasive à base de silice.

7. Bain de bouche comprenant du phosphate de zinc et un acide aminé basique sous forme libre ou sous la forme d'un sel acceptable par voie orale, dans lequel le phosphate de zinc est ajouté au bain de bouche sous la forme d'un sel préformé, et dans lequel
l'acide aminé basique est l'arginine en une quantité de 0,5 à 2 % en poids et le phosphate de zinc est ajouté en une quantité de 0,005 à 0,05 % en poids.

8. Bain de bouche selon la revendication 7, comprenant en outre une quantité efficace de : une source d'ions fluorure ; un ou plusieurs sels de phosphate alcalin ; un ou plusieurs agents antibactériens ; ou une combinaison de deux ou plus de ceux-ci.

9. Bain de bouche selon l'une quelconque des revendications 7 à 8, comprenant en outre : (i) un agent de blanchiment ; (ii) une ou plusieurs sources d'ion zinc en plus du phosphate de zinc ; ou une combinaison de ceux-ci.

10. Bain de bouche selon l'une quelconque des revendications 7 à 9, comprenant
0,1 à 2 % de phosphate de zinc ;
0,1 à 3 % d'arginine ;
2 à 8 % de sels de phosphate alcalin choisis parmi le phosphate dibasique de sodium, le phosphate dibasique de potassium, l'hydrogénophosphate de calcium dihydrate, le pyrophosphate de tétrasodium, le pyrophosphate de tétrapotassium, le pyrophosphate de calcium, le tripolyphosphate de sodium et des mélanges de deux ou plus de ceux-ci ;
700 à 2 000 ppm de fluorure.

11. Bain de bouche selon l'une quelconque des revendications 7 à 9, dans lequel la composition comprend
5 à 10 % de glycérine ;
3 à 7 % de sorbitol ;
5 à 10 % de propylène glycol ;
0 à 0,1 % TSPP ;
0,01 à 1 % d'édulcorant ;
0,01 à 1 % d'acide citrique ;
0 à 0,1 % de xanthane ;
0,005 à 0,05 % de phosphate de zinc ;
0 à 0,01 % de chlorure de cétyl pyridinium ;
0,01 à 0,1 % de sorbate de potassium ;
0,05 à 1 % d'arôme ; et
0,1 à 2 % de cocamidopropyl bétaïne.

12. Dentifrice selon l'une quelconque des revendications 1 à 6, ou le bain de bouche selon l'une quelconque des revendications 7 à 11, dans lequel le pH de la composition est acide.

13. Dentifrice selon l'une quelconque des revendications 1 à 6, ou le bain de bouche selon l'une quelconque des revendications 7 à 11, dans lequel le pH de la composition est basique.

14. Utilisation de phosphate de zinc et d'arginine sous forme libre ou sous la forme d'un sel acceptable par voie orale dans la fabrication d'un dentifrice ou d'un bain de bouche, dans laquelle le phosphate de zinc est ajouté au dentifrice sous la forme d'un sel préformé en une quantité de 0,5 à 4 % en poids, ou dans laquelle le phosphate de zinc est ajouté au bain de bouche sous la forme d'un sel préformé en une quantité de 0,005 à 0,05 % en poids, et dans laquelle l'arginine est présente dans le dentifrice ou le bain de bouche en une quantité de 0,5 à 10 % en poids.

15. Composition selon l'une quelconque des revendications 1 à 13, pour une utilisation dans le traitement ou la réduction de l'érosion de l'émail dentaire.
